# EUROPEAN PATENT APPLICATION

(11) **EP 3 290 508 A1**
(43) Date of publication of application: **07.03.2018**
(21) Application number: 16786123.6
(22) Date of filing: 15.04.2016
(51) Int. Cl.: C12M 3/00, C12M 1/00

(54) **CELL CULTURE DEVICE**

(30) Priority: 27.04.2015 JP 2015090633
(71) Applicant: Toyo Seikan Group Holdings, Ltd., Shinagawa-ku Tokyo 141-8627 (JP)
(72) Inventor: KOSEKI, Osamu, Tokyo 141-8627 (JP); SUENAGA, Ryo, Yokohama-shi Kanagawa 240-0062 (JP); KASHIWABARA, Ken, Yokohama-shi Kanagawa 240-0062 (JP)
(74) Representative: Liebetanz, Michael
(86) International application number: PCT/JP2016/002061
(87) International publication number: WO 2016/174844

(57) **Abstract**

Provided is a cell culture apparatus that can maintain communication between the inside and outside of the thermostatic chamber through a tube in a state where a door provided with a packing element being closed, and can automatically block communication of the inside and outside of the thermostatic chamber when the tube is removed. On an abutting surface (2a) around the opening part of the thermostatic chamber (3) and the cold storage chamber (4) that abuts against packing elements (3b, 4b) when front doors (3a, 4a) are closed, a communication groove (5) that intercommunicates the inside and outside of the thermostatic chamber (3) by the communication groove (51) when the transfer tube (U40) is removed is provided. The shut-off mechanism (5) is formed of: a guide hole (52) that opens on the abutting surface (2a) across the communication groove (51) and is deeper than the communication groove (51); a shutter member (53) which is inserted into the guide hole (52) and moves between the blocking position blocking the communication groove (51) and the opening position opening the communication groove (51); and a spring member (54) energizing the shutter member (53) to the blocking position.

## Description

### Technical Field

The present invention relates to a cell culture apparatus, more particularly, to a cell culture apparatus capable of communicating the inside and outside of a thermostatic chamber through a tube.

### Background Art

In recent years, it has been demanded to cultivate cells, tissues, etc. in an artificial environment efficiently in fields including production of pharmaceutical products, gene therapy, regenerative medicine, immunotherapy and the like.

In such cell culture, a cell culture unit in which a culture container used for cell culture and a culture medium container for supplying a culture medium to a culture container are connected through a tube is used. Since this cell culture unit is closed, cell culture can be conducted while eliminating the risk of contamination.

The following Patent Document 1 discloses a cell culture apparatus in which this type of cell culture units are stored in multistage.

### Related Art Document

### Patent Document

Patent Document 1: WO2007/052716

### Summary of the Invention

### Problems to be Solved by the Invention

In the cell culture, since control of temperature, air environment or the like is required, a culture container of the cell culture unit is arranged in a thermostatic chamber (incubator). On the other hand, since a culture medium of the cell culture unit is required to be stored in a refrigerator, a culture medium container is arranged in a cold storage chamber. Then, a culture medium is transferred from a culture medium container to a culture container through a tube.

The opening parts of the thermostatic chamber and the cold storage chamber of the cell culture apparatus are sealed by a door with a packing element in order to maintain the temperature or the gas concentration in the rooms. At this time, the culture container arranged in the thermostatic room and the cell culture medium container arranged in the cold storage chamber are kept connected through a tube. When the tube is caught in the door, the tube is crushed and it becomes difficult to transfer the culture medium through the tube. In order to avoid crush of the tube, a communication groove (concave portion) into which the tube can be fitted is formed on the abutting surface which can be in contact with the packing element of the door around the openings of the thermostatic chamber and the cold storage chamber.

By forming the communication groove on the abutting surface of the partition wall, not forming a communication hole in the partition wall, between the thermostatic chamber and the cold storage chamber, the culture container and the culture medium container can be arranged in the thermostatic chamber and the cold storage chamber, respectively, while being connected by a tube.

If such a communication groove is formed in a cell culture apparatus, the inside and outside of the thermostatic chamber and the cold storage chamber are intercommunicated when a tube is not fitted to the communication groove. In particular, in the cell culture apparatus in which cell culture units can be stored in multiple stages, communication grooves of unused stages in each of which no cell culture unit is arranged are kept intercommunicated. As a result, a carbon dioxide gas filled in the thermostatic chamber and heat are escaped from the communication groove, and such escape is not preferable from the viewpoint of the control of the temperature and the air environment of the thermostatic chamber.

If a dummy member is attached to the communication groove in which no tube is fitted, the communication groove can be blocked. However, it is troublesome to attach dummy members to the communication grooves one by one, and there is a risk of forgetting to attach a dummy member.

The present invention has been attained in view of the above-mentioned circumstances, and is aimed at providing a cell culture apparatus in which communication between the inside and outside of the thermostatic chamber through a tube is maintained in a state where a door with a packing element is closed, and communication of the inside and outside of the thermostatic chamber can be automatically blocked when the tube is removed.

### Means for Solving the Problem

In order to solve the problems mentioned above, the cell culture apparatus of the present invention is a cell culture apparatus provided with a thermostatic chamber of which an opening part is opened and closed by a door with a packing element, and on an abutting surface around the opening part that abuts against the packing element when the door is closed, a communication groove into which a tube that communicates the inside and outside of the thermostatic chamber to be fitted is formed, wherein the cell culture apparatus comprises a shut-off mechanism for blocking communication between the inside and outside of the thermostatic chamber through the communication groove when the tube is removed from the communication groove.

### Advantageous Effects of the Invention

According to the cell culture apparatus of the present invention, by forming a communication groove into which a tube that intercommunicates the inside and outside of the thermostatic chamber at an abutting surface around the opening of the thermostatic chamber to be fitted, not only communication of the inside and outside of the thermostatic chamber through a tube can be maintained in a state where a door with a packing element is closed, and by providing a shut-off mechanism, the communication of the inside and outside the thermostatic chamber through the communication groove can be automatically blocked when the tube is removed from the communication groove.

### Brief Description of the Drawings

Fig. 1 is a schematic view of an external appearance of the cell culture apparatus according to the embodiment of the present invention;
Fig. 2 is a schematic view of an external appearance of the cell culture apparatus according to the embodiment of the present invention;
Fig. 3 is an explanatory view showing one example of the cell culture unit stored in the cell culture apparatus according to the embodiment of the present invention;
Fig. 4 is an explanatory cross-sectional view of the inside of the thermostatic chamber of the cell culture apparatus according to the embodiment of the present invention;
Fig. 5 is an explanatory plan view of the cell culture unit arranged in the cell culture apparatus according to the embodiment of the present invention;
Fig. 6 is a perspective view of a block having shut-off mechanisms in the cell culture apparatus according the embodiment of the present invention;
Fig. 7 is a cross-sectional view taken along line A-A in Fig. 6 that shows a center pillar and doors of the cell culture apparatus according to the embodiment of the present invention, in which (a) shows a blocked state and (b) shows an intercommunicated state; and
Fig. 8 is a cross-sectional view taken along line B-B in Fig. 6, in which (a) shows a blocked state and (b) shows an intercommunicated state.

### Best Mode for Carrying out the Invention

Herein below, the embodiment of the present invention will be explained with reference to the drawings.

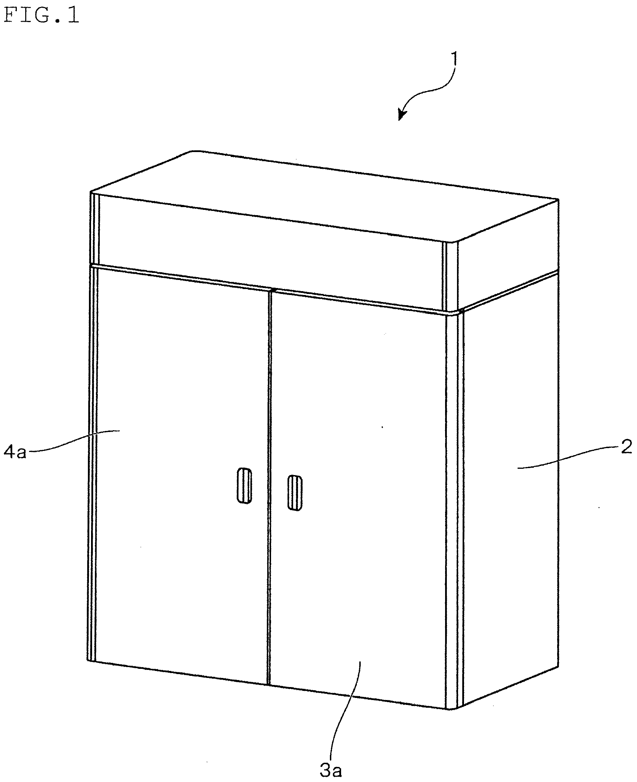

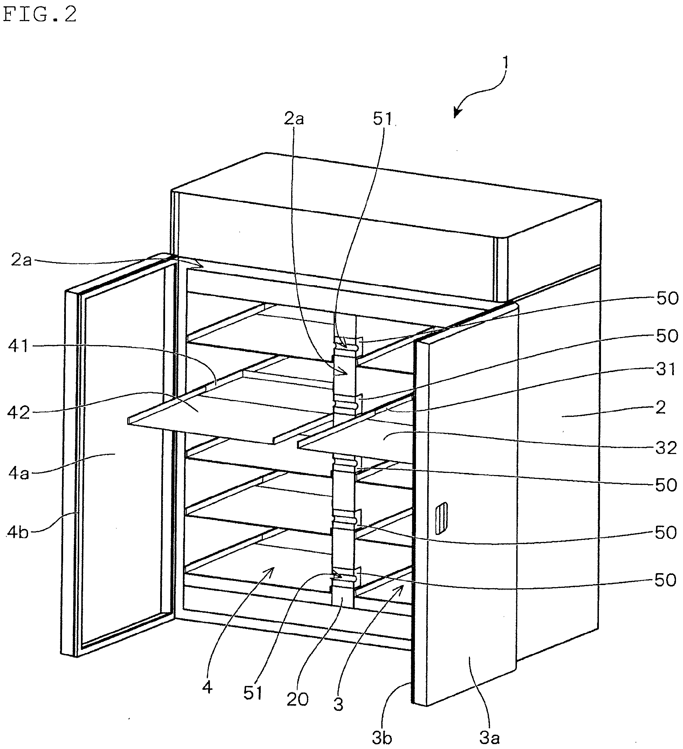

Figs. 1 and 2 show a schematic external view of the cell culture apparatus 1 of the present embodiment. The cell culture apparatus 1 is provided with the thermostatic chamber 3 and the cold storage chamber 4. They are formed by partitioning the inside of the housing 2 by a center pillar 20 of a heat insulating wall. The opening part of each of the thermostatic chamber 3 and the cold storage chamber 4 is closed and opened independently by a front door 3a and a front door 4a. The front door 3a and the front door 4a are provided with a packing element 3b and a packing element 4b, respectively. When the front door 3a and the front door 4a are closed, the packing element 3b and the packing element 4b abut against the abutting surface 2a of the casing 2 and the center pillar 20, whereby the thermostatic chamber 3 and the cold storage chamber 4 are each sealed. In the abutting surface 2a of the center pillar 20, a resin-made block 50 in which a communication groove 51 that intercommunicates the thermostatic chamber 3 and the cold storage chamber 4 is fitted.

The thermostatic chamber 3 is adjusted to have a temperature preferable for cell culture (e.g. 37°C), and is adjusted to have a gas concentration that keeps a prescribed carbon dioxide concentration (e.g. 5%). The cold storage chamber 4 is adjusted to have a temperature preferable for storage of a culture medium (e.g. 4°C).

Accommodation shells 32 and 42 are arranged for storing a plurality of cell culture units U in multistage in the thermostatic chamber 3 and the cold storage chamber 4. In order to facilitate the arrangement and removal of the cell culture units U, the accommodation shells 32 and 42 are configured such that they can be respectively drawn by slide rails 31 and 41.

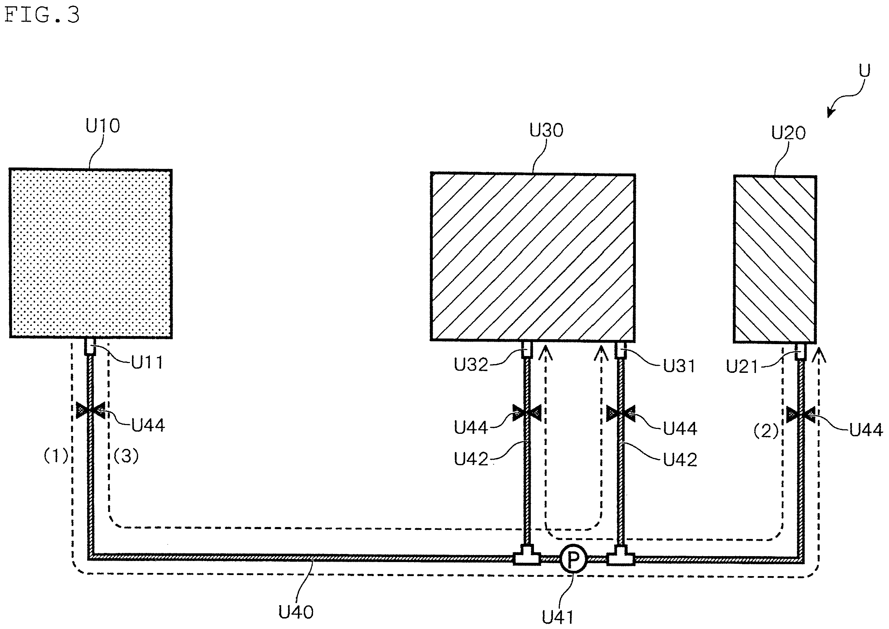

Fig. 3 shows one example of the cell culture unit U stored in the cell culture apparatus 1. The cell culture unit U comprises a first culture container U20 in which cell culture is conducted at first, a second culture container U30 to which a cell suspension is transferred after the cell culture in the first culture container U20, a culture medium container U10 that supplies a culture medium to these culture containers U20 and U30, and transfer tubes U40 and U42 that connect these containers U10, U20 and U30.

Connection of the containers by the transfer tubes U40 and U42 is controlled by a blocking element U44 such as a pinch valve. The transfer tubes U40 and U42 are formed of a flexible material such as silicone rubber and a soft vinyl chloride resin.

When cell culture is conducted in the cell culture unit U, at first, a culture medium is transferred through the transfer tube U40 by means of the pump U41 from a port U11 in the culture medium container U10 to a port U21 of the first culture container U20 into which cells have been injected (see an arrow in a broken line (1) in Fig. 3). Subsequently, after cell culture in the first culture container U20, a cell suspension is transferred from the port U21 in the first cell culture container U20 to a port U32 of the second culture container U30 through the transfer tubes U40 and U42 by means of the pump U41 (see an arrow in a broken line (2) in Fig. 3). Further, a culture medium is transferred from the port U11 in the culture medium container U10 to the port U31 of the second culture container U30 through the transfer tubes U40 and U42 by means of the pump U41 (see an arrow in a broken line (3) in Fig. 3).

As mentioned above, the culture medium is transferred through the transfer tube U40 from the culture medium container U10 arranged in the cold storage chamber 4 to the first and second culture containers U20 and U30 arranged in the thermostatic chamber 3. Therefore, when the front doors 3a and 4a of the cell culture apparatus 1 are closed, it is required to maintain communication through the transfer tube U40 between the thermostatic chamber 3 and the cold storage chamber 4.

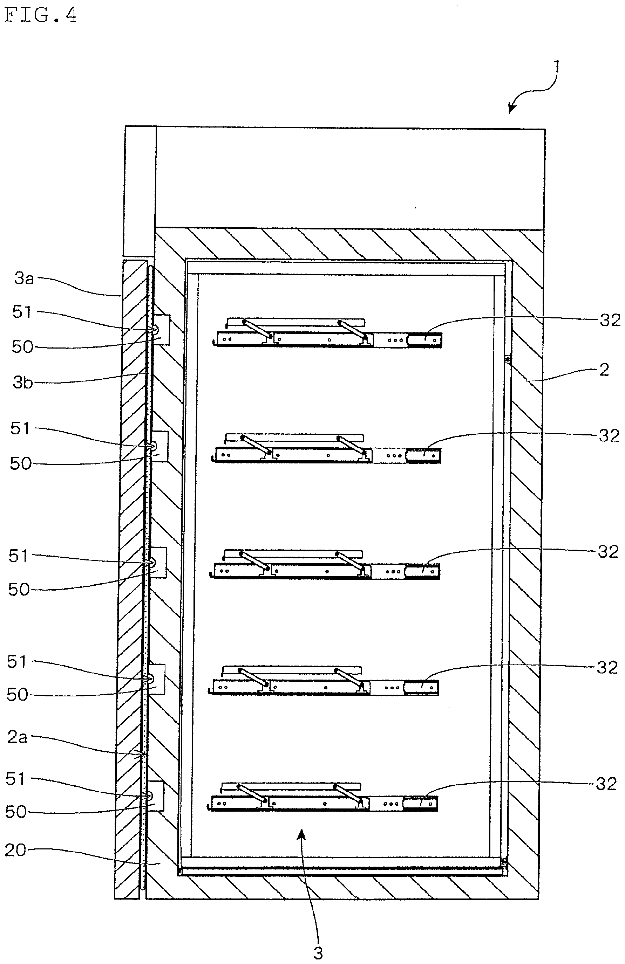

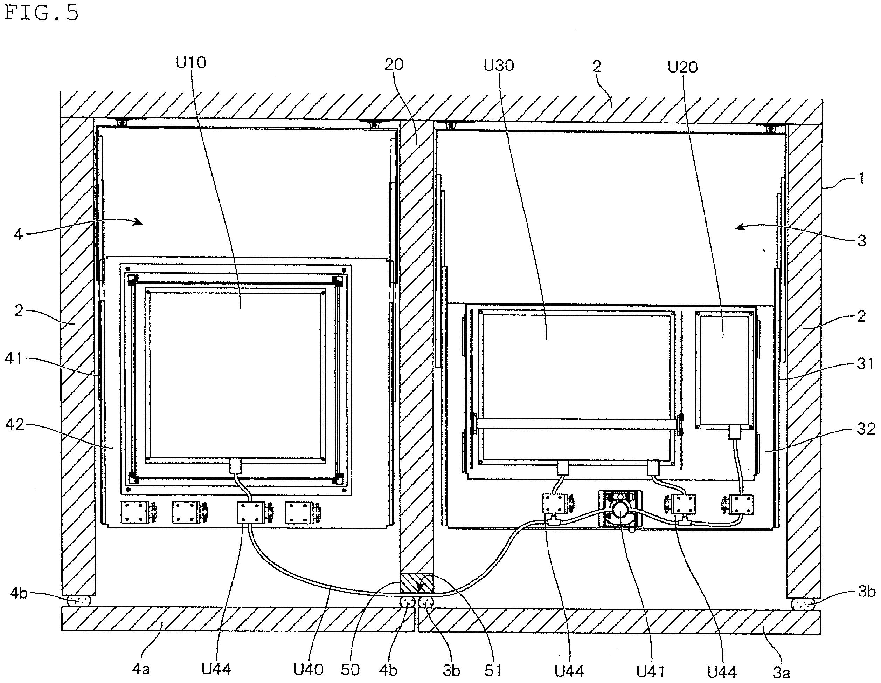

Figs. 4 and 5 respectively show the longitudinal cross-sectional view and the transverse cross-sectional view of the cell culture apparatus 1. Fig. 4 shows, for the purpose of convenience, the constitution inside the thermostatic chamber 3 in an overlapped manner on the cross section taken along the center pillar 20 in the cell culture apparatus 1. Fig. 5 shows the first and second culture containers U20 and U30 arranged on a culture container accommodation shelf 32 in the thermostatic chamber 3 and the culture medium container U10 arranged on a culture medium container storage shelf 42 in the cold storage chamber 4.

As shown in Figs. 4 and 5, when the front doors 3a and 4a are closed, the packing elements 3b and 4b abut against a housing 2 and an abutting surface 2a of the center pillar 20 of the cell culture apparatus 1. A resin-made block 50 is embedded in the abutting surface 2a of the center pillar 20 for each culture container accommodation shelf, and is fixed by a bolt (not shown). A communication groove 51 having almost the same width as that of the transfer tube U40 is formed in the block 50.

The communication groove 51 is formed so as to permit the thermostatic chamber 3 to communicate with the cold storage chamber 4, and the transfer tube U40 of the cell culture unit U is fitted into the communication groove 51. Therefore, as shown in Fig. 5, the transfer tube U40 is not crushed by the packing elements 3b and 4b when the front doors 3a and 4a are closed, whereby communication through the transfer tube U40 between the thermostatic chamber 3 and the cold storage chamber 4 is maintained.

If the thermostatic chamber 3 and the cold storage chamber 4 are kept communicated with the outside through the communication groove 51 when the transfer tube U40 is not fitted into the communication groove 51, heat and carbon dioxide gas are escaped from the thermostatic chamber 3 through the communication groove 51, and such escapes are not preferable in view of control of the temperature and air environment in the thermostatic chamber 3. Further, since external heat enters the cold storage chamber 4 through the communication groove 51, it is not preferable in view of temperature control in the cold storage chamber 4.

The cell culture apparatus 1 of the present embodiment is provided with a shut-off mechanism 5 for blocking communication of the thermostatic chamber 3 and the cold storage chamber 4 with the outside through the communication groove 51 when the transfer tube U40 is removed from the communication groove 51.

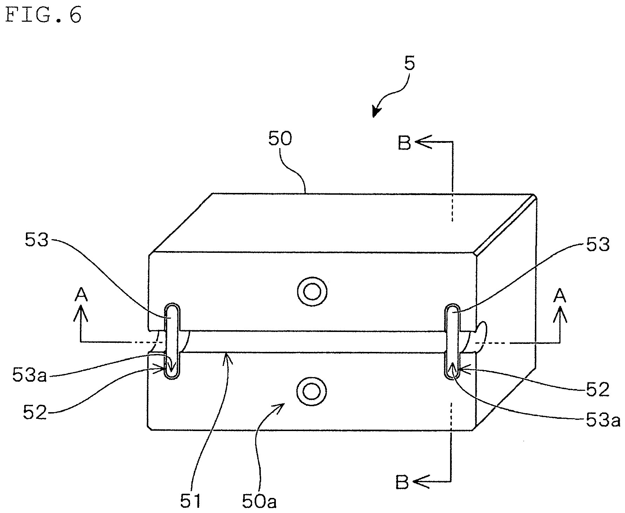

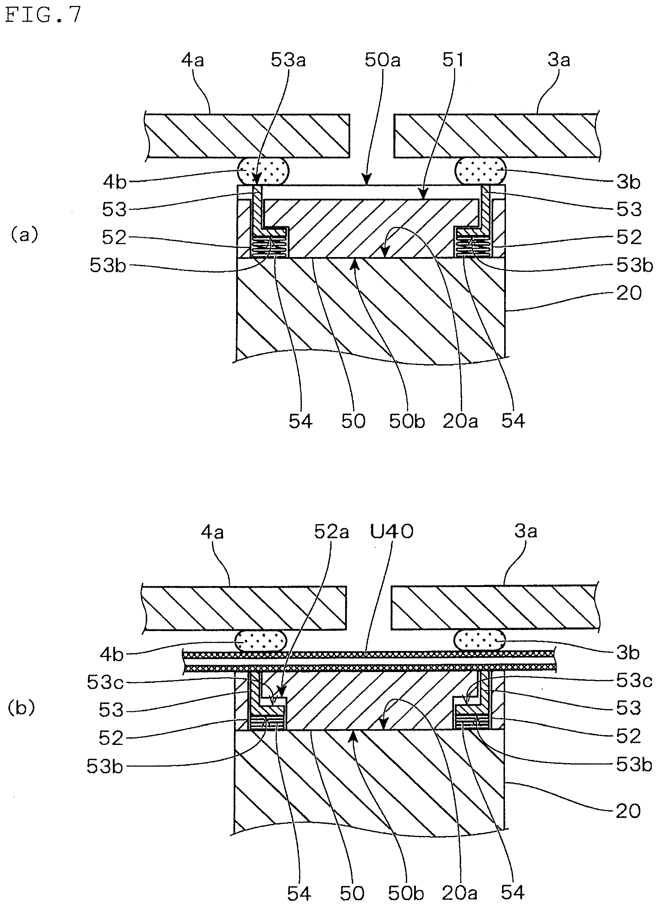

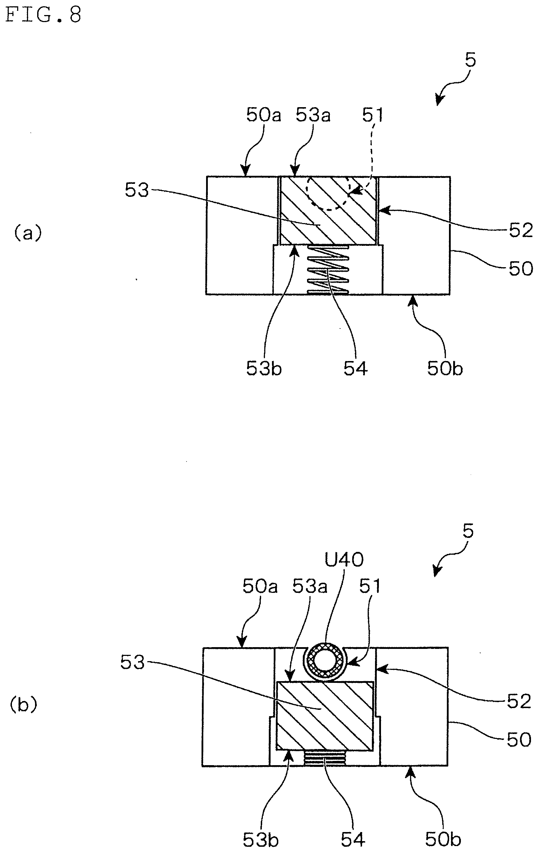

Fig. 6 shows a perspective view of the block 50 that constitutes the shut-off mechanism 5. Figs. 7 and 8 show the cross sectional views taken along the line A-A and B-B in Fig. 6, respectively. Fig. 7 shows the block 50 fitted in the center pillar 20 and the front doors 3a and 4a are closed.

An upper surface 50a of the block 50 on which the communication groove 51 is formed serves as the abutting surface 2a on which the packing elements 3b and 4b abut. Two guide holes 52 are formed in the block 50 so that each guide holes 52 opens on the upper surface 50a and crosses the communication groove 51. Each guide hole 52 is opened at a position in the abutting surface 2a where the position is covered by the packing elements 3b and 4b when the front doors 3a and 4a are closed. The guide hole 52 has larger depth than the communication groove 51. In the present embodiment, the guide hole 52 penetrates a bottom surface 50b of the block 50. The guide hole 52 may or may not necessarily penetrate.

A plate-like shutter member 53 that is bent in an L-shape and a coil-like spring member 54 are inserted in the guide hole 52. The shutter member 53 can move between a blocking position that blocks the communication groove 51 shown in Figs. 6, 7(a) and 8(a) and an opening position that opens the communication groove 51 shown in Figs. 7(b) and 8(b).

The upper part of the shutter member 53 blocks the communication groove 51 at the blocking position. On the other hand, the lower part 53b of the shutter member 53 is bent at right angle with respect to the upper part of the shutter member 53. The lower surface 53b of the lower part serves as a receiving part 53b receiving one end of the spring member 54. The other end of the spring member 54 abuts on the abutting surface 20a of a notched part of the center pillar 20. The spring member 54 serves as a press spring, and energizes the shutter member 53 to the blocking position.

The upper surface 53c of the lower part of the shutter member 53 serves as an abutting part 53c abutting on the inner surface 52a of the guide hole 52 when the shutter member 53 is energized to the blocking position. Due to the abutting of the abutting part 53c on the inner surface of the guide hole 52, the shutter member 53 is prevented from flying out of the opening part of the guide hole 52, and the upper end surface 53a of the shutter member 53 is flushed with the abutting surface 2a when the shutter member 53 is positioned at the blocking position.

As shown in Figs. 7(a) and 8(a), when the transfer tube U40 is not attached to the communication groove 51, the shutter member 53 energized by the spring member 54 is positioned at the blocking position. Then, as shown in Fig. 7(a), the upper end surfaces 53a of the two shutter members 53 abut against the packing elements 3b and 4b of the front doors 3a and 4a closed. As a result, the communication groove 51 is blocked by the two shutter members 53, whereby the thermostatic chamber 3 and the cold storage chamber 4 are sealed. The degree of the sealing may or may not necessarily be water tight.

As shown in Figs. 7(b) and 8(b), when the transfer tube U40 is fitted into the communication groove 51, the shutter member 53 is pushed down to the opening position by the tube U40. Preferably, the spring constant of the spring member 54 is small so that the transfer tube U40 can be fitted into the communication groove 51 without receiving a large resistance and so that the transfer tube U40 fitted into the communication groove 51 is not pushed out.

In Fig. 8(b), in order to allow the drawings to be understood easily, a gap is shown between the communication groove 51 and the outer periphery of the transfer tube U40. The gap is substantially not present, and the transfer tube U40 abuts against the upper end surface 53a of the shutter member 53, and pushes the shutter member 53 to the opening position.

As mentioned above, according to the cell culture apparatus of the present embodiment, communication between the thermostatic chamber 3 and the cold storage chamber 4 can be maintained with the front doors 3a and 4a provided with the packing elements 3b and 4b being closed, and when the transfer tube U40 is not attached to the communication groove 51, the communication groove 51 can be automatically blocked by the shutter member 53.

Hereinabove, the embodiment of the present invention was explained. The present invention is not restricted to the above-mentioned embodiment, and various modifications are possible within the scope of the present invention. For example, in the above-mentioned embodiment, an example is given in which the communication groove 51 is formed between the thermostatic chamber 3 and the cold storage chamber 4. The cell culture apparatus of the present invention is not restricted to one having a two-chamber structure of a thermostatic chamber and a cold storage chamber, and one which is not provided with a cold storage chamber can be applied. Further, a communication groove may be formed at any part of the abutting surfaces around the opening of the thermostatic chamber.

In the above-mentioned embodiment, an explanation was made on an example in which a linear communication groove 51 is formed. In the present invention, the shape of the communication groove is not limited thereto, and a winding communication groove, or a communication groove of which the width or the depth partially changes, may be formed.

In the above-mentioned embodiment, an explanation was made on an example in which the shutter member 53 is moved in a direction perpendicular to an abutting surface between the blocking position and the opening position. In the present invention, the shutter member may be moved in an oblique direction relative to the abutting surface or rotated. Further, in the above-mentioned embodiment, an explanation is made on an example in which the plate-like shutter member 53 is used. In the present invention, the shape of the shutter member is not restricted to this, and a shutter member having various shapes (e.g. cylindrical, prismatic, truncated cone, truncated pyramid, etc.) can be used.

In the above-mentioned embodiment, an explanation is made on an example in which a coil-like spring member 54 is provided. In the present invention, a spring member is not limited thereto. An appropriate and preferable energizing member such as a plate spring or various elastic bodies may be used.

In the above-mentioned embodiment, an explanation was made on an example in which, in the resin-made block 50 that can be processed easily, the communication groove 51 and the guide hole 52 are formed, and the shutter member 53 and the spring member 54 are incorporated into the block 50. In the present invention, the resin-made block may not necessarily be used, and a communication groove, etc. may be directly formed on the abutting surface of the center pillar or the housing, and a shutter member, etc. may be incorporated into the center pillar or the housing. The block may be made from resin or metal such as aluminum.

### Industrial Applicability

The present invention can preferably be used in regenerative medicine, immunotherapy, production of antibody medicine, etc. in which a large amount of cells is cultured in a closed system.

The documents described in the specification and the specification of the Japanese application on the basis of which the present application claims Paris convention priority are incorporated herein by reference in its entirety.

### Description of Numerical Symbols

1. Cell culture apparatus
2. Housing
2a Abutting surface
20. Center pillar
3. Thermostatic chamber
3a. Front door
3b. Packing element
31. Slide rail
32. Culture container accommodation shelf
4. Cold storage chamber
4a. Front door
4b. Packing element
41. Slide rail
42. Culture medium container accommodation shelf
5. Shut-off mechanism
50. Block
50a. Abutting surface
50b. Bottom surface
51. Communication groove
52. Guide hole
53. Shutter member
53a. Upper end surface
53b. Receiving part
53c. abutting part
54. Spring member
U. Cell culture unit
U10. Culture medium container
U20. First culture container
U30. Second culture container
U40. Transfer tube

## Claims

1. A cell culture apparatus provided with a thermostatic chamber of which an opening part is opened and closed by a door with a packing element and being formed a communication groove, into which a tube that communicates the inside and outside of the thermostatic chamber to be fitted, in an abutting surface around the opening part that abuts against the packing element when the door is closed, comprising
a shut-off mechanism for blocking communication between the inside and outside of the thermostatic chamber through the communication groove when the tube is removed from the communication groove.

2. The cell culture apparatus according to claim 1, wherein the shut-off mechanism comprises:
a guide hole which is opened in the abutting surface, cross the communication groove and has a larger depth than the communication groove;
a shutter member which is fitted into the guide hole and is displaced between the blocking position for blocking the communication groove and an opening position for opening the communication groove, and
a spring member that energizes the shutter member to the blocking position.

3. The cell culture apparatus according to claim 2, wherein the guide hole is opened at a position in the abutting surface that is covered by the packing element when the door is closed.

4. The cell culture apparatus according to claim 2 or 3, wherein the shutter member has an upper end surface that is flushed with the abutting surface when the shutter member is positioned on the blocking position.

5. The cell culture apparatus according to any one of claims 2 to 4, wherein the shutter member has a receiving part that receives the spring member and an abutting part that abuts against an inner surface of the guide hole when the shutter member is energized to the blocking position by the spring member.

6. The cell culture apparatus according to any one of claims 1 to 5, wherein the cell culture apparatus has a cold storage chamber adjacent to the thermostatic chamber, and the communication groove is formed on the abutting surface of a center pillar separating off the cold storage chamber from the thermostatic chamber.

7. The cell culture apparatus according to any one of claims 2 to 6, wherein the cell culture apparatus comprises a block fitted to the abutting surface,
the communication groove and the guide hole are formed in the block, and
the shutter member and the spring member are incorporated in the block.
